(19) Europäisches Patentamt
European Patent Office
Office européen des brevets

(11) Veröffentlichungsnummer: **0 254 225**
**A2**

(12) **EUROPÄISCHE PATENTANMELDUNG**

(21) Anmeldenummer: **87110366.9**

(22) Anmeldetag: **17.07.87**

(51) Int. Cl.[4]: **C07D 219/06 , A61K 31/435**

(30) Priorität: **22.07.86 DE 3624778**

(43) Veröffentlichungstag der Anmeldung: **27.01.88 Patentblatt 88/04**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Dhar, Rajkumar, Dr.**
**G-1, Hoechst Quarters Mulund (West)**
**Bombay 400 082(IN)**
Erfinder: **Desai, Premanand, Dr.**
**403, Sea-Side Apartments P. Balu Marg**
**Prabhadevi Bombay 400 025(IN)**
Erfinder: **Chatterjee, Dipak Kumar, Dr.**
**G-21, Hoechst Quarters Mulund (West)**
**Bombay 400 082(IN)**
Erfinder: **Rupp, Richard Helmut, Dr.**
**Niladri 66 Nepean Sea Road**
**Bombay 400 006(IN)**
Erfinder: **de Souza, Noel John, Dr.**
**Melrose 62 Pali Hill Bandra**
**Bombay 400 050(IN)**

(54) **Neue Derivate von 3-Aryl-7-chlor-3,4-dihydro-10-hydroxy-1,9(2H)-acridindionen ein Verfahren zu ihrer Herstellung und ihre Verwendung als Chemotherapeutika und Futterzusatz.**

(57) Die vorliegende Erfindung betrifft neue 3-Aryl-7-chlor-3,4-dihydro-10-hydroxy-1,9(2H,10H)-acridindionderivate, ein Verfahren zu ihrer Hestellung, sowie ihre Verwendung als Chemotherapeutika, insbesondere als Mittel gegen Malaria und Coccidiose.

Die erfindungsgemäßen Verbindungen können als Arzneimittel und als Zusatz zu Tierfuttermitteln verwendet werden.

EP 0 254 225 A2

**Neue Derivate von 3-Aryl-7-chlor-3,4-dihydro-10-hydroxy-1,9(2H)-acridindionen ein Verfahren zu ihrer Herstellung und ihre Verwendung als Chemotherapeutika und Futterzusatz**

Die vorliegende Erfindung betrifft neue 3-Aryl-7-chlor-3,4-dihydro-10-hydroxy-1,9(2H,10H)-acridindionderivate, ein Verfahren zu ihrer Herstellung, sowie ihre Verwendung als Chemotherapeutika, insbesondere als Mittel gegen Malaria und Coccidiose.

Die folgenden Patente und Veröffentlichungen betreffen 3-Aryl-7-chlor-3,4-dihydro-10-hydroxy-1,9 (2H,10H)-acridindion sowie seine Derivate:

1. Deutsche Patentschrift 23 37 474
2. Deutsche Offenlegungsschrift 27 48 333
3. Arzneimittelforschung, 30 (11), Nr. 7, S. 1041-46 (1980)
4. Eur.J.Med.Chem.-Chim.Ther. 20, Nr. 4 S. 363-370 (1985).

Verschiedene der im Stand der Technik beschriebenen Acridone sind wirksame Chemotherapeutika gegen Protozoeninfektionen.

Bei den erfindungsgemäßen Verbindungen handelt es sich um 10-Alkoxy-, 10-Carbalkoxy-und 10-Phenylsulfonyloxyderivate des 3-Aryl-7-chlor-3,4-dihydro-10-hydroxy-1,9(2H,10H )-acridindions. Die Verbindungen sind bis jetzt weder in der Literatur beschrieben noch nahegelegt worden. Lediglich die im Deutschen Patent DE 23 37 474 beschriebenen 10-Acyloxyderivate besitzen eine ähnliche Struktur wie die erfindungsgemäßen Verbindungen. Die erfindungsgemäßen Verbindungen unterscheiden sich von diesen Verbindungen darin, daß sie einen 3-Arylsubstituenten im Acridindionkern besitzen. Weiterhin besitzen die erfindungsgemäßen Verbindungen eine stärkere Wirksamkeit gegen Erreger der Malaria als die besten der bisher beschriebenen Verbindungen, wie beispielsweise das Floxacrin-(7-chlor-3,4-dihydro-10-hydroxy-3-(4-trifluormethylphenyl)-1,9-(2 H, 10H)-acridindion).

Die neuen 10-Hydroxyderivate des 3-Aryl-7-chlor-3,4-dihydro-10-hydroxy-1,9(2H ,10H)-acridindions werden durch die Formel I wiedergegeben:

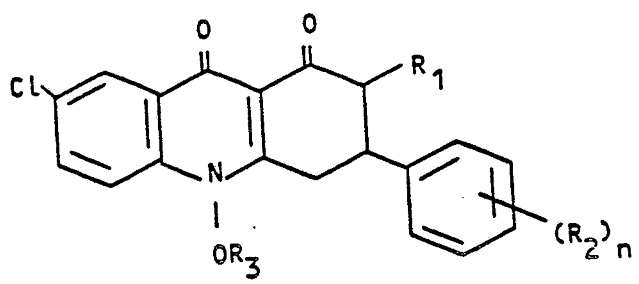

(I)

worin

$R_1$ Wasserstoff, $C_1$-$C_4$-Alkyl, $C_2$-$C_5$-Carbalkoxy oder Phenyl welches ein-oder mehrfach mit $C_1$-$C_4$-Alkyl, Halogen oder Amino substituiert sein kann, bedeutet,

$R_2$ Halogen oder Trifluormethyl bedeutet, wobei die Substituenten, falls n eine Zahl von 2 oder 3 bedeutet, gleich oder verschieden sein können

n eine ganze Zahl von 0 bis 3, und

$R_3$ $C_1$-$C_4$ Alkyl, Carbalkoxy oder Phenylsulfonyl bedeutet, worin die Phenylgruppe gegebenenfalls ein oder mehrfach mit $C_1$-$C_4$-Alkyl, Halogen oder Amino substituiert sein kann.

Falls $R_1$ und $R_3$ für eine Alkylgruppe stehen, handelt es sich dabei um Methyl, Äthyl, Propyl, Isopropyl oder Butyl.

Halogen bedeutet Chlor, Brom, Fluor oder Jod.

Falls $R_1$ eine Carbalkoxygruppe bedeutet, sind darunter bevorzugt eine Carbmethoxy-oder eine Carbäthoxygruppe zu verstehen.

Die Arylgruppe steht bevorzugt für eine gegebenenfalls substituierte Phenylgruppe.

Die erfindungsgemäßen Verbindungen sind neue Derivate, die wertvolle Chemotherapeutika zur Prophylaxe und Behandlung der Malaria darstellen.

Bevorzugte erfindungsgemäße Verbindungen werden durch die Formel I dargestellt, worin $R_1$, $R_2$ und $R_3$ die obigen Bedeutungen besitzen.

Die folgenden Verbindungen sind besonders bevorzugt:

7-Chlor-3,4-dihydro-10-methoxy-3-(4-trifluormethylphenyl)-1,9(2H,10H)-acridindion,

7-Chlor-3,4-dihydro-10-methoxy-2-methyl-3-(4-trifluormethylphenyl)-1,9(2H,10H)-acridindion,

7-Chlor-3,4-dihydro-10-äthoxy-3-(4-trifluormethylphenyl)-1,9(2H,10H)-acridindion,

10-(4-Aminophenylsulfonxyloxy)-7-chlor-3,4-dihydro-3-(4-trifluormethylphenyl)-1,92 H,10H)-acridindion.

In der nachfolgenden Tabelle sind einige erfindungsgemäße Verbindungen aufgeführt:

Tabelle 1

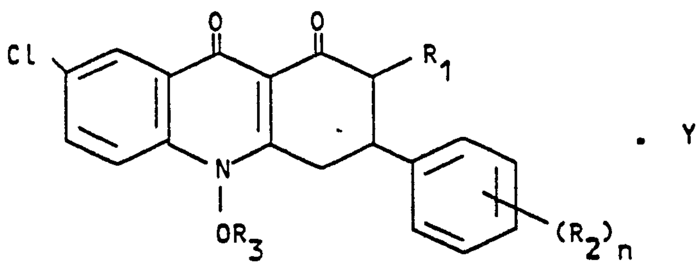

| $R_1$ | $R_2$ | $R_3$ | Y | Schmelzpunkt °C |
|---|---|---|---|---|
| H | Cl | $CH_3$ | $H_2O$ | > 300 |
| H | $CF_3$ | $CH_3$ | | > 300 |
| H | Cl | $C_2H_5$ | $H_2O$ | > 250 |
| H | $CF_3$ | $C_2H_5$ | | > 290 |
| H | $CF_3$ | $C_3H_7$ | | > 200 |
| H | $CF_3$ | $C_4H_9$ | | > 300 |
| $CH_3$ | $CF_3$ | $CH_3$ | | 287-89 |
| H | $CF_3$ | $COOC_2H_5$ | $0.5H_2O$ | 246 (Zers.) |
| H | $CF_3$ | $SO_2-C_6H_4-p-CH_3$ | | 258-9 |
| H | $CF_3$ | $SO_2C_6H_4-p-Cl$ | | 206-7 |
| H | $CF_3$ | $SO_2C_6H_4-p-NH_2$ | $H_2O$ | 248 (Zers.) |

Die vorliegende Erfindung betrifft weiterhin ein Verfahren zur Herstellung der Verbindungen der Formel I, dadurch gekennzeichnet, daß man ein entsprechend substituiertes 7-Chlor-10-hydroxyacridindion der allgemeinen Formel II

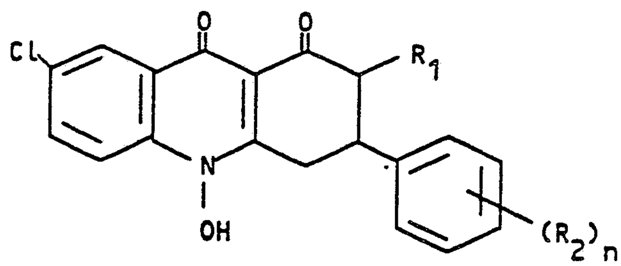

(II)

worin $R_1$ und $R_2$ die obigen Bedeutungen besitzen, in Gegenwart einer Base, wie beispielsweise Kaliumcarbonat oder Kalium-t-butoxyd mit einem geeigneten Alkylhalogenid, Dialkylsulfat oder Arylsulfonylhalogenid in einem wasserfreien Lösungsmittel, wie t-Butanol oder Toluol, zweckmäßigerweise bei Zimmertemperatur, 1 bis 24 Stunden lang umsetzt.

Die als Ausgangsverbindungen verwendeten Acridindione der Formel II können gemäß dem im deutschen Patent Nr. 23 37 474 beschriebenen Verfahren, wie aus dem nachfolgenden Reaktionsschema ersichtlich

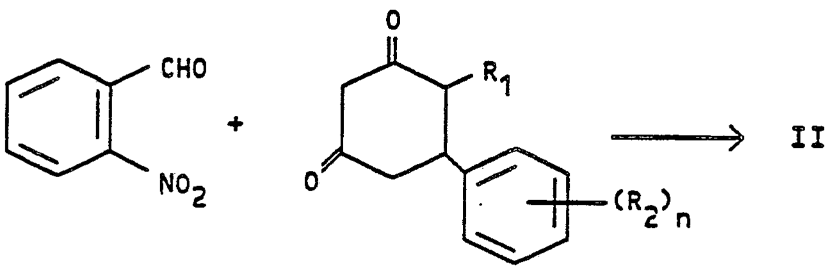

worin $R_1$, $R_2$ und n die obigen Bedeutungen haben, hergestellt werden.

Die erfindungsgemäßen Verbindungen sind farblose bis gelbe kirstalline Verbindungen, die wenig löslich in Wasser sind und meistens bei Temperaturen über 200°C schmelzen. Sie können säulenschromatographisch oder durch Kristallisation aus Lösungsmitteln, wie Methanol, Chloroform, Dimethylformamid usw. gereinigt werden.

Die erfindungsgemäßen Verbindungen besitzen wertvolle chemotherapeutische Eigenschaften bei der Bekämpfung von Protozoeninfektionen. So zeichnen sie sich beispielsweise durch eine hohe Wirksamkeit gegen Plasmodia, die Erreger der Malaria, aus. Weiterhin sind sie wie in einem entsprechenden Modell gezeigt werden kann, wirksam gegen den Chlorochin-resistenten Stamm Plasmodium berghei. So wird nach Verabreichung der erfindungsgemäßen Verbindungen an durch Plasmodium berghei infizierten Mäusen in Dosen von 10-25 mg/kg x 5 eine vollständige Ausheilung der Parasitämie erreicht.

Die Verbindungen der Formel I können oral oder parenteral in Dosen von 2,5 bis 100 mg/kg Körpergewicht verabreicht werden. Wenn sie bei der Bekämpfung der Malaria eingesetzt werden, zieht man Einheitsdosisformen wie Dragees oder Kapseln zur oralen Verabreichung oder Lösungen oder Suspensionen zur Injektion mit jeweils 100 bis 400 mg Wirkstoff vor. Diese Dosiseinheiten werden ein-bis dreimal täglich, je nach Verfassung des Patienten, verabreicht.

Die vorliegende Erfindung betrifft ferner eine pharmazeutische Zubereitung, die eine erfindungsgemäße Verbindung in Mischung oder Verbindung mit einem pharmazeutisch geeigneten Träger enthält. Die Zubereitung kann in einer für die orale oder parenterale Verabreichung geeigneten Form, vorzugsweise in Einheitsdosisformen, vorliegen.

Zur oralen Verabreichung eignen sich Tabletten, Dragees, Kapseln, Pulver oder Granula, die den Wirkstoff in Mischung oder Verbindung mit Substanzen, wie beispielsweise Stärke, Cellulosepulver, Talk, Magnesiumstearat, Zucker, Gelatine, Calciummcarbonat, fein zerteilte Kieselsäure oder Carboxymethylcellulose enthält.

Zur parenteralen Verabreichung, insbesondere zu intramuskulären Injektionen, eignen sich sterile Suspensionen, beispielsweise ölige Suspensionen, die unter Verwendung von Sesamöl, Rizinusöl oer synthetischen Triglyzeriden, gegebenenfalls unter gleichzeitiger Verwendung von oberflächenaktiven Substanzen, wie Sorbitanfettsäureestern, hergestellt werden. Weiterhin eignen sich auch wäßrige Suspensionen, die beispielsweise unter Verwendung von äthoxylierten Sorbitan-Fettsäureestern gegebenenfalls unter Zugabe von Verdickungsmitteln, wie beispielsweise Polyethylenglykol oder Carboxymethylcellulose, hergestellt werden.

Weiterhin sind die erfindungsgemäßen Verbindungen wirksam gegen die Erreger der Coccidiose, beispielsweise die Hühner-, Truthahn-, Kaninchen-, Rinder-und Schweinecoccidiose. Bei den heutigen Aufzuchtsmethoden für Schlachttiere stellt die Coccidiose ein schweres Problem während der Aufzuchts- und Mastperiode dar, da sie beträchtliche wirtschaftliche Verluste verursacht. So ist die Verfügbarkeit von hoch wirksam, gut verträglichen Coccidiostatika von großem Interesse.

Zu diesem Zweck können die Verbindungen der Formel I mit geeigneten Futtermitteln gemischt verabreicht werden. Demzufolge betrifft die Erfindung ebenfalls ein modifiziertes Tierfutter, das eine Verbindung der Formel I in Mischung mit einem Futtermittel enthält.

Die Erfindung wird durch die nachfolgenden Beispiele erläutert:

Herstellungsbeispiele:

Beispiel 1:

7-Chlor-3,4-dihydro-10-methoxy-3-(4-trifluormethylphenyl)-1,9(2H,10H)-acridindion

Eine Suspension aus 7-Chlor-3,4-dihydro-10-methoxy-3-(4-trifluormethylphenyl)1,9(2H,10H)-acridindion (4,07 g) in wasserfreiem t-Butanol wurde bei Zimmertemperatur gerührt und dann mit einer Lösung aus Kalium-t-butoxyd in t-Butanol (hergestellt aus 1 g metallischem Kalium und 25 ml t-Butanol) versetzt. Das gelbe Reaktionsgemisch wurde 30 Minuten lang gerührt und mit Methyljodid (20 ml) versetzt. Das erhaltene Reaktionsgemisch wurde 8 Stunden gerührt, in Eiswasser gegossen, filtriert und der Rückstand mit Methanol gewaschen und getrocknet, wobei man 7-Chlor-3,4-dihydro-10-methoxy-3-(4-trifluormethylphenyl)-1,9(2H,10H)-acridindion (3,2 g; 90,8 %), Fp > 300°C, erhielt.

Verfährt man analog obigem Verfahren, jedoch unter Verwendung von Äthyljodid anstelle von Methyljodid, so erhält man die Verbindung 7-Chlor-3,4-dihydro-10-äthoxy-3-(4-trifluormethylphenyl)-1,9(2H,10H)-acridindionmonohydrat, Fp. > 250°C.

Verfährt man analog dem in Beispiel 1 beschriebenen Verfahren, jedoch unter Verwendung von 7-Chlor-3,4-dihydro-10-hydroxy-2-methyl-3-(4-trifluormethylphenyl)-1,9(2H,10H)-acridindion anstelle von 7-Chlor-3,4-dihydro-10-hydroxy-3-(4-trifluormethylphenyl)-1,9-(2H,10H)-acridindion, so erhält man die Verbindung 7-Chlor-3,4-dihydro-10-methoxy-2-methyl-3-(4-trifluormethylphenyl)-1,9(2H,10H)-acridindion, Fp. 287-289°C.

Beispiel 2:

10-(4-Aminophenylsulfonyloxy)-7-chlor-3,4-dihydro-3-(4-trifluormethylphenyl)1,9(2 H, 10H)-acridinidion-monohydrat

Eine Suspension aus 7-Chlor-3,4-dihydro-10-hydroxy-3-(4-trifluormethylphenyl)-1,9(2H,10H)-acridindion (1,06 g) und p-Acetaminophenylsulfonylchlorid (0,98 g) in trockenem Methanol wurde 15 Stunden lang gerührt. Dann engte man die erhaltene klare Lösung ein und ließ sie stehen, wobei sich Kristalle des 10-(4-Aminophenylsulfonyloxy)-7-chlor-3,4-dihydro-3-(4-trifluormethylphenyl)-1,9(2 H, 10H)-acridindionmonohydrats (0,648 g), Fp. 248°C (Zers.) absetzten.

**Ansprüche**

1. Verbindungen der Formel I

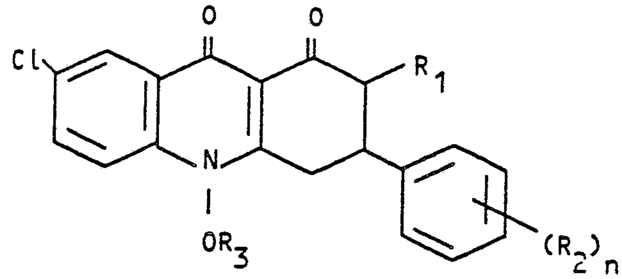

(I)

worin bedeuten:

$R_1$ Wasserstoff, $C_1$-$C_4$-Alkyl, $C_2$-$C_5$-Carbalkoxy oder Phenyl, welches ein-oder mehrfach mit $C_1$-$C_4$-Alkyl, Halogen oder Amino substituiert sein kann,

$R_2$ Halogen oder Trifluoromethyl, wobei die Substituenten, falls n eine Zahl von 2 oder 3 bedeutet, gleich oder verschieden sein können,

n eine ganze zahl von 0 bis 3

$R_3$ $C_1$-$C_4$-Alkyl, $C_2$-$C_3$-Carbalkoxy oder die Phenylsulfonyl-Gruppe, wobei der Phenylkern ein oder mehrfach mit $C_1$-$C_4$-Alkyl, Halogen oder Amino substituiert sein kann.

2. Verbindungen der Formel I gemäß Anspruch 1, worin

$R_1$ Wasserstoff oder Methyl

$R_2$ Fluor, Chlor oder Trifluormethyl,

n die Zahl 1,

$R_3$ $C_1$-$C_4$-Alkyl, $C_2$-$C_5$-Carbalkoxy oder eine Phenylsulfonylgruppe, worin der Phenylrest einfach mit Methyl, Halogen oder Amino substituiert sein kann,

bedeutet.

3. Verfahren zur Herstellung einer Verbindung gemäß Anspruch 1, dadurch gekennzeichnet, daß man eine Verbindung der Formel II

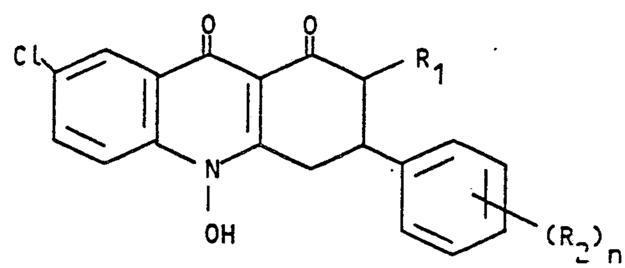

(II)

worin $R_1$, $R_2$ und n die genannten Bedeutungen haben, mit einer Verbindung der Formel $R_3$ Hal, worin $R_3$ die obengenannte Bedeutung hat und Hal ein Halogenatom bedeutet, in Gegenwart einer Base in einem wasserfreien organischen Lösungsmittel bei Raumptemperatur umsetzt.

4. Verfahren gemäß Anspruch 3, dadurch gekennzeichnet, daß als Base Kaliumcarbonat oder Kalium-tert.-butoxid verwendet wird.

5. Arzneimittel, gekennzeichnet durch einen Gehalt an einer Verbindung gemäß Anspruch 1 und pharmazeutisch üblichen Hilfs-und/oder Trägerstoffen.

6. Futterzusatz, gekennzeichnet durch einen Gehalt an einer Verbindung gemäß Anspruch 1 als Wirkstoff.

7. Verwendung einer Verbindung gemäß Anspruch 1 zur Herstellung von Arzneimitteln mit Wirksamkeit gegen Protozoen-Infektionen.

8. Verwendung einer Verbindung gemäß Anspruch 1 zur Herstellung von Arzneimitteln mit Wirksamkeit gegen Malaria.

9. Verwendung einer Verbindung gemäß Anspruch 1 als Futtermittelzusatz zur Verhinderung und Bekämpfung von Coccidose bei Nutztieren.

Patentansprüche für folgende Vertraggsstaaten: AT und ES.

1. Verfahren zur Herstellung einer Verbindung der Formel I

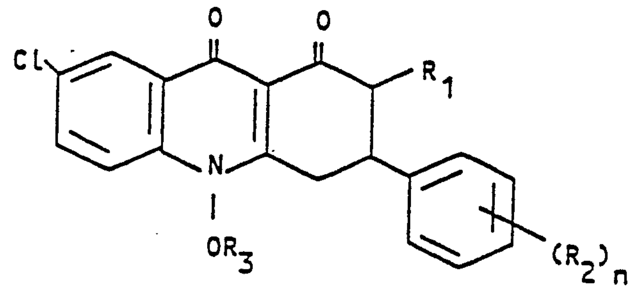

(I)

worin bedeuten:

$R_1$ Wasserstoff, $C_1$-$C_4$-Alkyl, $C_2$-$C_5$-Carbalkoxy oder Phenyl, welches ein-oder mehrfach mit $C_1$-$C_4$-Alkyl, Halogen oder Amino substituiert sein kann,

$R_2$ Halogen oder Trifluoromethyl, wobei die Substituenten, falls n eine Zahl von 2 oder 3 bedeutet, gleich oder verschieden sein können,

n eine ganze zahl von 0 bis 3

$R_3$ $C_1$-$C_4$-Alkyl, $C_2$-$C_3$-Carbalkoxy oder die Phenylsulfonyl-Gruppe, wobei der Phenylkern ein oder mehrfach mit $C_1$-$C_4$-Alkyl, Halogen oder Amino substituiert sein kann, dadurch gekennzeichnet, daß man eine Verbindung der Formel II

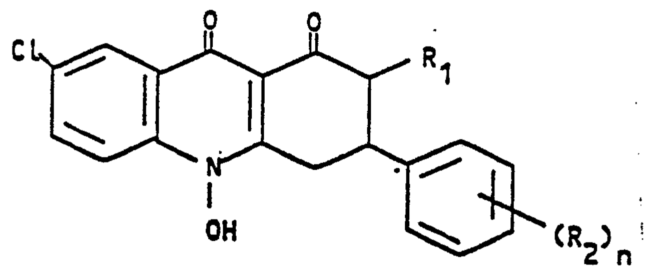

(II)

worin $R_1$, $R_2$ und n die genannten Bedeutungen haben, mit einer Verbindung der Formel $R_3$ Hal, worin $R_3$ die obengenannte Bedeutung hat und Hal ein Halogenatom bedeutet, in Gegenwart einer Base in einem wasserfreien organischen Lösungsmittel bei Raumtemperatur umsetzt.

2. Verfahren nach Anspruch 1, dadurch gekennzeichnet, daß man eine Verbindung der Formel I herstellt, worin

$R_1$ Wasserstoff oder Methyl

$R_2$ Fluor, Chlor oder Trifluormethyl,

n die Zahl 1,

$R_3$ $C_1$-$C_4$-Alkyl, $C_2$-$C_5$-Carbalkoxy oder eine Phenylsulfonylgruppe, worin der Phenylrest einfach mit Methyl, Halogen oder Amino substituiert sein kann, bedeutet.

3. Verfahren gemäß Anspruch 1 und 2, dadurch gekennzeichnet, daß als Base Kaliumcarbonat oder Kalium-tert-.butoxid verwendet wird.

4. Arzneimittel, gekennzeichnet, durch einen Gehalt an einer Verbindung gemäß Anspruch 1 und pharmazeutisch üblichen Hilfs-und/oder Trägerstoffen.

5. Futterzusatz, gekennzeichnet durch einen Gehalt an einer Verbindung gemäß Anspruch 1 als Wirkstoff.

6. Verwendung einer Verbindung gemäß Anspruch 1 zur Herstellung von Arzneimitteln mit Wirksamkeit gegen Protozoen-Infektionen.

7. Verwendung einer Verbindung gemäß Anspruch 1 zur Herstellung von Arzneimitteln mit Wirksamkeit gegen Malaria.

8. Verwendung einer Verbindung gemäß Anspruch 1 als Futtermittelzusatz zur Verhinderung und Bekämpfung von Coccidiose.